# EUROPEAN PATENT APPLICATION

(11) **EP 4 763 122 A1**
(43) Date of publication of application: **24.06.2026**
(21) Application number: 25224274.8
(22) Date of filing: 17.12.2025
(51) Int. Cl.: A61B 34/20

(54) **AUTOMATED DETECTION OF A DEPLOYMENT STATE OF A CATHETER THROUGH A SHEATH**

(30) Priority: 18.12.2024 US 202418986304
(71) Applicant: Biosense Webster (Israel) Ltd., 2066717 Yokneam (IL)
(72) Inventor: GLINER, Vadim, 2066717 Yokneam (IL)
(74) Representative: Carpmaels & Ransford LLP

(57) **Abstract**

A method includes measuring an impedance of an electrode coupled to a catheter as the electrode is delivered through a delivery sheath of the catheter inside a blood pool of a cardiac chamber. Steepest slope of the impedance is identified. The impedance at steepest slope is defined as a threshold value. A user is provided with at least one of a first indication that the electrode is in the sheath as long as the impedance is greater than the threshold and a second indication that the electrode is at least partially out of the sheath as long as the impedance is lower than the threshold.

## Description

### FIELD OF THE DISCLOSURE

The present disclosure relates generally to invasive medical probes, and particularly to identification of a deployment state of a distal end of a catheter delivered through sheath.

### BACKGROUND OF THE DISCLOSURE

Techniques to determine a deployment of a catheter distal end through its delivery sheath were previously proposed in patent literature. For example, U.S. Patent 10,398,347 describes catheterization that is carried out by inserting a sheath into a human patient and moving a catheter having an electrode through the sheath lumen. A variation between a first threshold value and a second threshold value in electrical current through the electrode is identified. Responsively to the variation, it is reported that a portion of the catheter has transitioned between an in-sheath condition and an out-of-sheath condition. The sheath is defined and identified by the historical data of a readings of the magnetic sensor of the catheter during its movements.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electroanatomical (EA) mapping and ablation system, according to an example of the present disclosure;
Fig. 2 is a schematic graph of a catheter's electrode impedance as a function of electrode location relative to the distal edge of the delivery sheath of Fig. 1, according to an example of the present disclosure; and
Fig. 3 is a flow chart schematically illustrating a method and algorithm to detect when each electrode, or the entire distal end assembly, of the catheter is fully in, exiting, or fully out of the sheath, according to an example of the present disclosure.

### DETAILED DESCRIPTION OF EXAMPLES

### OVERVIEW

Diagnostic and therapeutic catheters are typically delivered through a sheath. It is desirable to identify a deployment state of the distal end assembly of the catheter through the sheath before using the catheter for clinical tasks. For example, it would be desirable to verify that an electrode is emerging out of the sheath and/or when the electrode is fully outside the sheath prior to performing electro-anatomical (EA) mapping or ablation with that electrode. It may also be desirable to verify when a catheter or an electrode of a catheter is fully retracted inside the sheath.

A physician who operates the catheter may receive feedback such as a visualization of the status of one or more of the electrodes on the catheter during catheter deployment. For example, an electrode may be marked black on a display of the catheter-based system while the electrode is inside the delivery sheath and white while the electrode is outside the delivery sheath.

To ensure proper clinical application of the catheter, the indication of the deployment state of the distal end assembly of the catheter and/or the deployment state of each electrode on the distal end assembly through the sheath should be robust, regardless of sheath type (e.g., diameter) and catheter type (e.g., tip, lasso, among others).

One possible solution for assessing the deployment state of an electrode of the catheter through a sheath is to measure the electrical impedance between the electrode and an electrode attached to the skin. A measured impedance is expected to be significantly higher while inside the sheath as compared to when it is fully deployed. For example, the impedance measured deep inside the sheath may be in the order of magnitude of about 10,000 ohms, while the impedance outside the sheath may be in the order of magnitude of 100 ohms. A threshold on the impedance may be defined to identify when the electrode emerges out of the sheath, e.g., to differentiate between when an electrode is deployed from the sheath and retracted into the sheath. For example, the electrode impedance may be compared to a pre-defined electrode impedance threshold defined during a calibration procedure and stored in memory.

However, the same catheter may be introduced through different-sized sheaths. Likewise, different-sized catheters may be used with the same sheath. The impedance measured is sensitive to the diameter of the sheath, the size of the catheter, and the size of the electrodes. Using one threshold for call combinations, per catheter and/or per sheath can yield false indications. One threshold does not provide enough sensitivity to the different combinations.

Examples of the present disclosure that are described hereinafter dynamically define the impedance threshold during the clinical procedure. The disclosed technique uses measured impedance characteristics of one or more electrodes of the catheter to automatically identify when each electrode (or the entire distal end assembly) is either in or out of the sheath.

In addition to being highly reliable, the disclosed technique is applicable to many sheath and catheter types. The method relies on analyzing properties of the characteristics of the impedance curve of electrode impedance as a function of distance from the distal edge (exit point) of a sheath. Evaluation of multiple types of catheters and sheaths has revealed that the impedance curve tends to have a universal characteristic of an inverse sigmoid.

In one example, the system circuitry measures a catheter electrode impedance as the electrode is delivered (e.g., moved) through a delivery sheath (e.g., as a function of the electrode displacement through a delivery sheath) inside a blood pool of a cardiac chamber. The physical location of the catheter is obtained using an independent tool, such as magnetic location sensing or intracardiac ultrasound imaging. This step may be performed during a calibration procedure (in which the sheath and catheter are immersed in fluid that mimics the blood pool), and/or at the beginning stage of the clinical procedure.

A system processor identifies the steepest slope of the inverse sigmoid impedance curve as a function of the displacement to define a steepest-slope related threshold value. In an exmaple, a processor identifies the steepest slope based on detecting a minimum (or maximum) in the impedance curve derivative (e.g., using a second derivative of the original curve). The slope-related threshold value is the impedance measured at the steepest slope. steepest The processor applies the steepest-slope-related threshold value during a clinical procedure to identify when the distal-end assembly of the catheter is outside the sheath.

The catheter electrode impedance curve can be measured multiple times by the catheter being advanced and retracted (e.g., re-entering) through the delivery sheath. In one example, the measurement, identification, and definition of the steepest-slope-related threshold value can start with that of a distal electrode. Using this threshold value, the measurement, identification, and definition of the steepest-slope-related threshold value for a proximal electrode are then done and refined, and the processor proceeds to apply this steepest-slope-related threshold value to the proximal electrode.

Each electrode can preferably have its own threshold to indicate when the electrode (or the entire distal end assembly) is fully in or out of the sheath or use one dynamic threshold for all the electrodes on the catheter.

The dynamic threshold definition process can be initiated based on determining a significant change in impedance measured. At a start of a procedure, the measured impedance may hover around a value. An average and standard deviation of the value may be determined based on accumulated data. The process of computing the slope for the purpose of identifying the steepest negative slope may be initiated once the impedance changes significantly (e.g., drops). The process may be initiated or updated once the processor identifies that the impedance change exceeds a threshold change or by identifying an absolute change above a given amount or several standard deviations (STD) based on accumulated impedance sensed over a given period.

In another example, the processor measures impedance as a function of time and looks for the steepest slope. This method saves the need to consider position explicitly. In any method used herein the steepest slope indicates implicitly that the electrode is located about at the edge of the sheath without quantifying the relative location.

Finally, the processor displays a virtual representation of the catheter and alters the color (or another graphical encoding, such as pattern) of the electrode based on the impedance being greater or lower than the threshold.

### SYSTEM DESCRIPTION

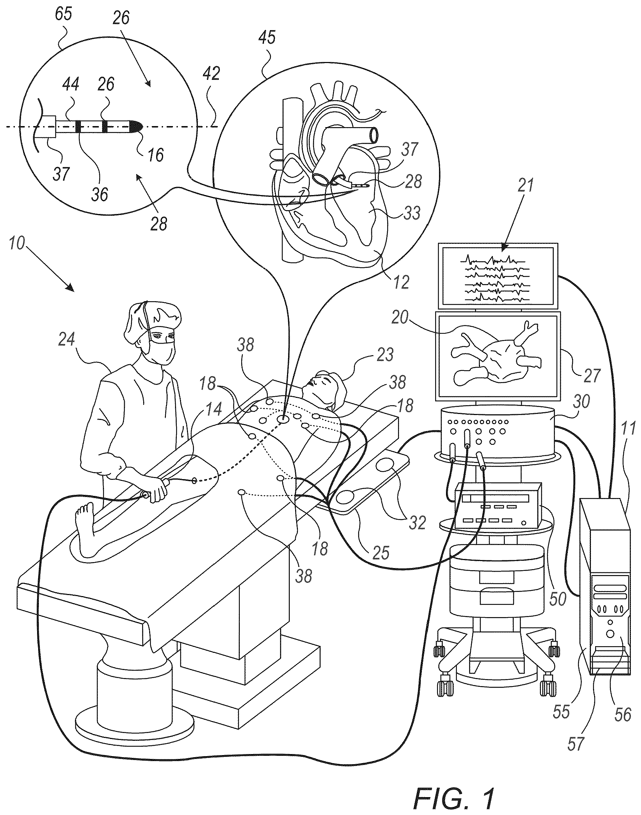

Fig. 1 is a schematic, pictorial illustration of a catheter-based electro-anatomical (EA) mapping and ablation system 10, according to an example of the present disclosure. System 10 includes a delivery sheath 37 (illustrated in insets 45 and 65) percutaneously inserted by a physician 24 through the patient's vascular system into a chamber or vascular structure of a heart 12. Thereafter, physician 24 deploys a tip catheter 14 of system 10 in a chamber of a heart 12 (e.g., in a blood pool of a ventricle 33).

In Fig. 1, physician 24 advances a tip distal-end assembly 28 fitted on a shaft 44 of catheter 14 into contact with the heart wall for EA sensing a target site in heart 12 or for ablating the wall tissue. Assembly 28 has a longitudinal axis 42, which is parallel to a distal end of shaft 44. The terms "proximal" and "distal" are defined in relation to position over that axis.

As seen in inset 65, tip assembly 28 includes a tip electrode 16 and ring electrodes 26 and 36. Electrode 16 may sense unipolar EA signals or may be used for radiofrequency (RF) ablation. Electrodes 16, 26, and 36 may be used for sensing bipolar EA signals, for electrical position tracking of tip assembly 28, and pulsed-field ablation (PFA).

System 10 further includes one or more electrode patches 38 positioned for skin contact on patient 23 to enable (i) sensing the unipolar EA signals (e.g., between any of electrodes 16, 26, and 36, and a common electrode realized by one or more patches 38), and (ii) impedance at electrodes 16, 26, and 36, e.g., between electrodes 16, 26, and 36, and one or more electrode patches 38.

A recorder 11 displays electrograms 21 captured with body surface ECG electrodes 18 and EA signals captured with electrodes 16, 26 and 36 of catheter 14.

System 10 may include an ablation energy generator 50 adapted to conduct electrical ablative energy to electrode 16 at the distal assembly 28 of catheter 14 configured for electrical ablation. Energy produced by ablation energy generator 50 may include, but is not limited to, radiofrequency (RF) energy or pulsed-field ablation (PFA) energy, including monopolar or bipolar high-voltage DC pulses (or combinations thereof) that may be used to effect irreversible electroporation (IRE).

A patient interface unit (PIU) 30 is configured to establish electrical communication between catheter 14, electrophysiological equipment, power supply, and a workstation 55 for controlling the operation of system 10. Electrophysiological equipment of system 10 may include, for example, other catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

Workstation 55 includes memory 57, a processor 56 with memory or storage with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded electrograms 21 in representative visual indicia or imagery superimposed on the rendered anatomical map 20, (iii) displaying real-time location and orientation of multiple catheters within the heart chamber, and (iv) displaying on display device 27 sites of interest such as places where ablation energy has been applied. One commercial product embodying elements of system 10 is available as the CARTO^{™} 3 System, available from Biosense Webster, Inc., 31A Technology Drive, Irvine, CA 92618.

While Fig. 1 describes a tip catheter, the description hereinafter applies to other catheter types, such as loop, multi-arm, and basket multi-electrode self-expanding catheters, which are fitted with one or more ring electrodes, such as electrodes 26 and 36, on a distal end portion of the shaft of the catheter (i.e., proximally to the self-expandable assembly).

### AUTOMATIC SHEATH TYPE DETECTION

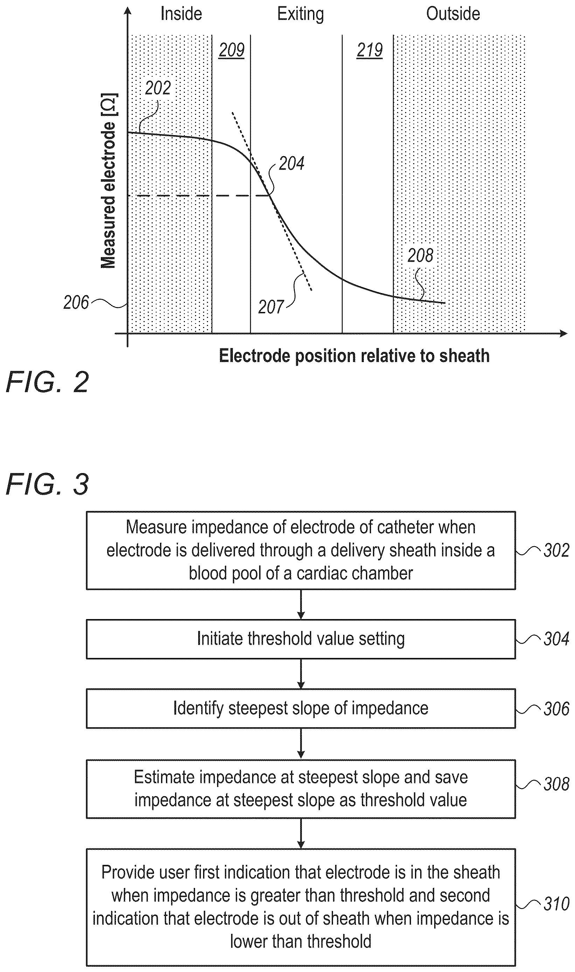

Fig. 2 is a schematic graph of a catheter's electrode impedance as a function of electrode location relative to the distal edge of sheath 37 of Fig. 1, according to an example of the present disclosure. The schematic graph applies to any of electrodes 16, 26, and 36, while the actual value may vary between electrodes. This graph applies to different catheter types and sheath types, while the actual value may vary between catheters and sheaths. As noted above, the impedance of an electrode is measured between that electrode and a suitable reference electrode. If Fig. 1, for example, an electrode patch 38 may serve as a reference electrode for such impedance measurements.

As Fig. 2 shows, electrode impedance drops along a characteristic inverse sigmoid curve from a value of 202 inside the sheath to a value of 208 outside the sheath. The impedance changes significantly at one of the regions 209 and 219 of the impedance curve. The significant change can be quantified as being one more than a given amount or several standard deviations (STD). While values 202 and 208 may vary, as explained above, the inverse sigmoid shape nevertheless remains robust.

In particular, the steepest slope 207 of the inverse sigmoid curve occurs close to a center point between values 202 and 208.

Using the disclosed algorithm, processor 56 identifies maximum slope 207 of the impedance for one or more of electrodes 16, 26, and 36. Processor 56 defines a steepest slope-related threshold value as either impedance 206 measured at the steepest slope steepest).

The definitions for values 206 and/or 207 are robust enough to allow for automatic detection when each electrode, or the entire distal end assembly, of a catheter is in or out of the sheath. Optionally, the automated detection also differentiates between fully in, emerging, or fully out of the sheath for many types of catheters and sheaths.

### METHOD OF AUTOMATIC DETECTION OF CATHETER ELECTRODE LOCATION RELATIVE TO ANY SHEATH TYPE

Fig. 3 is a flow chart schematically illustrating a method and algorithm to detect when each electrode (16, 26, 36), or the entire distal end assembly 28, of catheter 14 emerges out of delivery sheath 37, according to an example of the present disclosure. Optionally, a default threshold is stored in memory and applied until an updated threshold is determined. The algorithm, according to the present example, carries out a process that begins at a signal measuring step 302, with interface 30 circuitries measuring signals (e.g., impedances) of one or more of electrodes 16, 26, and 36 of assembly 28 as it is displaced (e.g., advanced or retracted) through sheath 37 inside blood pool of an atrium or ventricle 33. This step takes place before the diagnostic/treatment steps of the clinical procedure.

At threshold-setting initiating step 304, the threshold-setting process is initiated or updated once the processor identifies a significant change in impedance. The significant change may be defined as an absolute pre-defined change or as a change beyond a defined number of standard deviations (STD) of measured values detected while the catheter is at one of the regions 209 and 219 of the impedance curve.

At identification step 306, processor 56 identifies a steepest negative slope 207 of the impedance related to one or more of electrodes 16, 26, and 36 emerging out of the sheath, as shown in Fig. 2. Additionally, processor 56 may also identify a steepest positive slope, e.g., slope 207 of impedance related to the one or more electrodes being retracted back into the sheath. This identification may be initiated after detecting that the one or more electrodes are already outside the sheath.

At an impedance estimation step 308, processor 56 estimates the impedance value 206 at the steepest negative slope 207. Processor 56 saves impedance value 206 as the threshold recorded at the steepest slope. The definition is made for one or more electrodes 16, 26, and 36. Typically the process is applied to at least proximal electrode 36.

During the clinical procedure, at threshold value application step 310, processor 56 provides to a user, at least one of a first indication that the electrode is in sheath 37 if the impedance is greater than the threshold 205 and a second indication that the electrode is at least partially (e.g., emerging) out of sheath 37 if the impedance is lower than the threshold 206.

Processor 56 uses the steepest slope-related threshold value, preferably that of proximal electrode 36, to check the distal end assembly 28 location relative to sheath 37, for example, to verify that distal end assembly 28 is exiting the sheath or located entirely outside sheath 37.

The example flow chart shown in Fig. 3 is simplified for conceptual clarity. For example, additional steps like multiple advancement and retractions of the catheter to obtain multiple measurements may also be applied. The threshold-setting process can be alternatively initiated or updated once the processor identifies that the impedance has changed more than a given amount or several standard deviations (STD). For example, threshold setting can start once impedance passes the shaded area in Fig. 2, indicating either increased exposure to an external environment, e.g., left shaded area 209, or decreased exposure to an external environment, e.g., right shaded area 219.

### EXAMPLES

### Example 1

A method includes measuring (302) an impedance of an electrode (16, 26, 36) coupled to a catheter (14) as the electrode is delivered through a delivery sheath (37) of the catheter inside a blood pool of a cardiac chamber (33). Steepest slope (207) of the impedance is identified. The impedance at steepest slope is defined as a threshold value (206). A user is provided with at least one of a first indication that the electrode (16, 26, 36) is in the sheath (37) as long as the impedance is greater than the threshold (206) and a second indication that the electrode (16, 26, 36) is at least partially out of the sheath (37) as long as the impedance is lower than the threshold (206).

### Example 2

The method according to example 1, wherein providing the indications to the user comprises displaying virtual representation of the catheter and altering a color if the electrode (16, 26, 36) based on the impedance being greater or lower than the threshold (206).

### Example 3

The method according to any of examples 1 and 2, wherein measuring the impedance of the electrode (16, 26, 36) as the electrode is delivered through the delivery sheath (37) comprises measuring the impedance as a function of displacement of the electrode through the delivery sheath.

### Example 4

The method according to any of examples 1 through 3, wherein measuring the impedance of the electrode (16, 26, 36) comprises individually measuring the impedances of more than one electrode and defining a threshold impedance (206) per electrode.

### Example 5

The method according to any of examples 1 through 3, wherein measuring the impedance of an electrode (16, 26, 36) comprises individually measuring the impedances of more than one electrode and defining a single threshold impedance (206) for a plurality of the electrodes (16, 26, 36).

### Example 6

The method according to any of examples 1 through 5, and comprising one of initiating threshold (206) setting process or updating the threshold upon identifying that a pre-defined change (209, 219) in impedance as compared to an average impedance measured over a defined time period.

### Example 7

The method according to any of examples 1 through 6, wherein the pre-defined change (209, 219) is defined as a pre-defined number of STD, wherein the STD is defined based on the impedance measured over the defined time period.

### Example 8

The method according to any of examples 1 through 7, and comprising updating the threshold (206) based on detecting re-entry of the electrode (16, 26, 36) into the sheath (37).

### Example 9

The method according to any of examples 1 through 8, wherein applying the steepest-slope (207) related threshold value (206) comprises identifying that a distal-end assembly (28) of the catheter (14) is outside the delivery sheath (37) by identifying that all electrodes (16, 26, 36) of the catheter are outside the delivery sheath.

### Example 10

The method according to any of examples 1 through 9, and comprising:
measuring the impedance, identifying the steepest slope (207) and defining the steepest-slope related threshold value for a distal electrode (16, 26);
using the steepest-slope related threshold value for the distal electrode (16, 26), measuring the impedance, identifying the steepest slope and defining the steepest-slope related threshold value for a proximal electrode (36) ; and
during the clinical procedure, applying the steepest-slope related threshold value (206) defined for the proximal electrode (36).

### Example 11

The method according to any of examples 1 through 10, and comprising measuring the displacement using one of magnetic location sensing (25) and intracardiac ultrasound imaging.

### Example 12

The method according to any of examples 1 through 11, and comprising tracking a position of the sheath (37) and determining a location of a distal end (28) of the catheter relative to the edge of the sheath (37).

### Example 13

A system (10) includes an interface (30) and a processor (56). The interface configured to measure an impedance of an electrode (16, 26, 36) coupled to a catheter (14) as the electrode is delivered through a delivery sheath (37) of the catheter inside a blood pool of a cardiac chamber (33); The processor is configured to (i) identify steepest slope (207) of the impedance, (ii) define the impedance at steepest slope as a threshold value (206), and (iii) provide to a user, at least one of a first indication that the electrode (16, 26, 36) is in the sheath (37) as long as the impedance is greater than the threshold (206) and a second indication that the electrode (16, 26, 36) is at least partially out of the sheath (37) as long as the impedance is lower than the threshold (206).

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A system (10), comprising:
an interface (30) configured to measure an impedance of an electrode (16, 26, 36) coupled to a catheter (14) as the electrode is delivered through a delivery sheath (37) of the catheter inside a blood pool of a cardiac chamber (33); and
a processor (56), which is configured to:
identify steepest slope (207) of the impedance;
define the impedance at steepest slope as a threshold value (206); and
provide to a user, at least one of a first indication that the electrode (16, 26, 36) is in the sheath (37) as long as the impedance is greater than the threshold (206) and a second indication that the electrode (16, 26, 36) is at least partially out of the sheath (37) as long as the impedance is lower than the threshold (206).

2. The system according to claim 1, wherein the processor is configured to provide the indications to the user by displaying virtual representation of the catheter and altering a color if the electrode (16, 26, 36) based on the impedance being greater or lower than the threshold (206).

3. The system according to any of claims 1 and 2, wherein the interface is configured to measure the impedance of the electrode (16, 26, 36) as the electrode is delivered through the delivery sheath (37) by measuring the impedance as a function of displacement of the electrode through the delivery sheath.

4. The system according to any of claims 1 through 3, wherein the interface is configured to measure the impedance of the electrode (16, 26, 36) by individually measuring the impedances of more than one electrode and defining a threshold impedance (206) per electrode.

5. The system according to any of claims 1 through 3, wherein the interface is configured to measure the impedance of an electrode (16, 26, 36) by individually measuring the impedances of more than one electrode and defining a single threshold impedance (206) for a plurality of the electrodes (16, 26, 36).

6. The system according to any of claims 1 through 5, wherein the processor is further configured to initiate threshold (206) setting process or update the threshold upon identifying that a pre-defined change (209, 219) in impedance as compared to an average impedance measured over a defined time period.

7. The system according to any of claims 1 through 6, wherein the pre-defined change (209, 219) is defined as a pre-defined number of STD, wherein the STD is defined based on the impedance measured over the defined time period.

8. The system according to any of claims 1 through 7, wherein the processor is further configured to update the threshold (206) based on detecting re-entry of the electrode (16, 26, 36) into the sheath (37).

9. The system according to any of claims 1 through 8, wherein the processor is configured to apply the steepest-slope (207) related threshold value (206) by identifying that a distal-end assembly (28) of the catheter (14) is outside the delivery sheath (37) by identifying that all electrodes (16, 26, 36) of the catheter are outside the delivery sheath.

10. The system according to any of claims 1 through 9, wherein the processor is further configured to:
identify the steepest slope (207) and defining the steepest-slope related threshold value for a distal electrode (16, 26);
using the steepest-slope related threshold value for the distal electrode (16, 26), identify the steepest slope and defining the steepest-slope related threshold value for a proximal electrode (36); and
during the clinical procedure, apply the steepest-slope related threshold value (206) defined for the proximal electrode (36).

11. The system according to any of claims 1 through 10, wherein the processor is further configured to measure the displacement using one of magnetic location sensing (25) and intracardiac ultrasound imaging.

12. The system according to any of claims 1 through 11, wherein the processor is further configured to track a position of the sheath (37) and determine a location of a distal end (28) of the catheter relative to the edge of the sheath (37).
